# EUROPEAN PATENT APPLICATION

(11) **EP 0 531 978 A2**
(43) Date of publication of application: **17.03.1993**
(21) Application number: 92115436.5
(22) Date of filing: 09.09.1992
(51) Int. Cl.: A61K 35/80, A61K 7/48

(54) **Promotor for biological hyaluronic acid synthesis, composition externally applied to the skin and use thereof**

(30) Priority: 10.09.1991 JP 258568/91
(71) Applicant: LION CORPORATION, Sumida-ku Tokyo (JP)
(72) Inventor: Hirayama, Yutaka, Lion Corporation, Ninomiya-machi, Naka-gun, Kanagawa-ken (JP); Ebata, Keiko, Naka-gun, Kanagawa-ken (JP); Watanabe, Shinichi, Atsugi-shi, Kanagawa-ken (JP)
(74) Representative: VOSSIUS & PARTNER

(57) **Abstract**

A promotor for biological hyaluronic acid synthesis and a composition for external application to the skin comprise, as an effective component, an extract of the seaweed Ulva pertusa. The promotor exhibits a substantially excellent biological hyaluronic acid synthesis-promoting effect and accordingly activates the skin cells, prevents the aging thereof and the formation of wrinkles and makes the skin smooth and fresh. Thus, it can provide a highly safe agent externally applied to the skin. Consequently, the composition can widely be used in a variety of preparations such as various cosmetic creams, milky lotions, toilet waters, beauty essences, packs, under-makeups, foundations, jellies and ointments.

## Description

The present invention relates to a promotor for biological hyaluronic acid synthesis which comprises an extract of plants, green algae, which is highly safe. It also relates to a composition to be externally applied to the skin which comprises the foregoing promotor, such as cosmetic creams and milky lotions, which can prevent the aging of the skin and which can make the skin wrinkle-free, smooth, moist and fresh.

Hyaluronic acid is widely distributed in living bodies such as in the skin, ligament, synovia and vitreous body and plays an important role, for instance, in the protection of cells, transfer of nutrients, retention of water and flexibility of tissues of the skin; and retention of the structure and functions of the tissues and retention of lubricating ability for the synovia.

On the other hand, if the cell functions are lowered due to aging and pathologic conditions of the skin and joint, the amount of such biological hyaluronic acid is decreased. This results in drying of the skin, skin chapping, reduction of tension and resiliency of the skin, increase of spots and wrinkles, or arthralgia or the like due to the reduction of lubrication of the joints.

There have been adopted, for improvement of these conditions, a method which comprises applying to the skin a cosmetic containing biological components such as hyaluronic acid and natural moisturizing factors and a method which comprises directly injecting hyaluronic acid into the joint, but the external application of these substances does not result in the complete improvement of the functions and a sufficient effect would not be achieved. In particular, hyaluronic acid is scarcely absorbed through the skin. For this reason, there has been a desire for the development of substances capable of basically improving functions of the living body by promoting the ability of hyaluronic acid synthesis of cells per se while making use of the self recuperative power which is innate in the living body, instead of externally supplementing the foregoing substances.

Hyaluronic acid is produced in, for instance, epidermal basal cells, fibroblasts and synovial cells. The hyaluronic acid produced in the epidermal basal cells is involved in the improvement in the water retention, protection of cells, supply of nutrients and acceleration of discharge of waste materials and accordingly, plays an important role in the maintenance of the health of the skin, in the improvement of the tension and complexion thereof and in making the texture of the skin fine. Therefore, a promoter for hyaluronic acid synthesis in the epidermal cells is quite effective for maintaining the health of the skin and has become of major interest as a wrinkle-improving agent lately. There has been known that retinoic acid shows such an effect, but has side-effects such as strong irritation of the skin and thickening of the skin. For this reason, it cannot be used as an effective ingredient for common drugs, quasi-drugs and cosmetics. Therefore, there has been a desire for the development of effective components which can basically improve the functions of the skin and are highly safe.

Accordingly, a primary object of the present invention is to provide a promotor which exhibits an excellent effect in promoting the biological synthesis of hyaluronic acid and is highly safe.

Another object of the present invention is to provide a composition for external application to the skin comprising the promoter and an inert carrier and/or diluent.

Another object of the present invention is to provide a use of the promoter and the composition.

These and other objects will be clear from the following description and Examples.

The inventors of this invention have conducted screenings of various plant's extracts while using the hyaluronic acid synthesis-promoting effect thereof on the epidermal cell of rats as an indication whether the foregoing problems are solved. They have found that an extract from a specific plant belonging to the genus Ulva of the family Ulvaceae exhibits the desired effect.

On the other hand, this effect is not detected in the conventionally known extracts from seaweeds, for instance, brown algae (Phaeophyceae) such as the genera Laminaria, Sargassum and Ecklonia; and green algae (Chlorophyceae) such as the genus Monostroma. The effect is peculiar to the extract of Ulva pertusa which is a plant belonging to the genus Ulva of the family Ulvaceae.

The promotor for biological hyaluronic acid synthesis according to the present invention comprises, as an effective component, an extract of Ulva pertusa which is a seaweed belonging to the genus Ulva of the family Ulvaceae.

Fig. 1 is a graph on which the radioactivity of every fraction of a culture medium (free of Ulva pertusa extract: control) is plotted (Example 1).

Fig. 2 is a graph on which the radioactivity of every fraction of a culture medium obtained in the presence of Ulva pertusa extract (500 µg/ml) is plotted (Example 1).

Fig. 3 is a photograph which shows the condition of the epidermis treated with a hydrophilic ointment containing Ulva pertusa extract.

Fig. 4 is a photograph which shows the condition of the epidermis treated with a control hydrophilic ointment containing no Ulva pertusa extract.

The Ulva pertusa which is a seaweed belonging to the genus Ulva of the family Ulvaceae usable in the invention has a membranous body and a large and/or small holes are formed therein as it grows. This was eaten during World War II by sprinkling it on boiled rice after it had been dried and finely pulverized. Ulva pertusa grows on rocks existing in the intertidal zone and is widely distributed in various coasts of the country.

Upon preparing the extract of the present invention, Ulva pertusa may be subjected to extraction without drying, but preferably it is extracted after drying by any known manner such as air-drying or lyophilization from the viewpoint of extraction efficiency.

The extract used in the invention may be prepared by a usual extraction method and can be obtained by extracting Ulva pertusa with water, a hydrophilic organic solvent, a water-containing hydrophilic organic solvent or other organic solvents.

Examples of hydrophilic organic solvents include lower alcohols such as methanol, ethanol and isopropanol, acetone, methyl ethyl ketone, acetonitrile, dimethylsulfoxide and dimethylformamide.

In particular, the plants are preferably extracted with water or a mixture of water and a lower alcohol such as methanol, ethanol or isopropanol. In this respect, the ratio of the lower alcohol to water is preferably 0/100 to 70/30 (v/v; volume ratio).

The ratio of the dried Ulva pertusa to the extraction solvent preferably ranges from 1/50 to 1/2.

Regarding other conditions for the extraction, the extraction temperature is not critical, but preferably ranges from 5 to 80 °C. The extraction is carried out at a temperature defined above for preferably 1 to 24 hours with stirring. In addition, the pH for the extraction is not critical as long as it is not extremely alkaline or acidic.

The residue remaining after the foregoing extraction process may further be repeatedly subjected to the foregoing extraction process for improving the extraction efficiency.

It is particularly preferred that the extracts obtained according to the foregoing extraction process be further subjected to a physical process such as the ultrafiltration and gel filtration or a combination thereof to give a fraction comprising components having molecular weights of not less than 3,000, a fraction comprising components having molecular weights of not more than 100,000 or a fraction comprising components having molecular weights falling within the range of 3,000 to 100,000.

The extract may be used as such or may be diluted, concentrated into a conc. extract, converted into dry powder through, for instance, lyophilization or formed into a paste-like product.

If the extract is converted into dry powder, the powder is preferably dissolved in water or a water-containing lower alcohol such as methanol, ethanol or isopropanol in advance, or solubilized in a water-containing composition for external application as will be detailed below, prior to use.

The promotor for biological hyaluronic acid synthesis according to the present invention exhibits an excellent effect of promoting biological synthesis of hyaluronic acid and is highly safe. Therefore, the promotor can be used in a variety of applications such as drugs, quasi-drugs and cosmetics.

If the promotor is used as an ingredient of a composition for external application to the skin, the extract of Ulva pertusa as an essential component may be incorporated into the composition in any concentration together with an inert carrier and/or diluent, but various compositions for external application to the skin may in general comprise the extract in an amount ranging from 0.01 to 30% by weight (hereinafter simply referred to as "%"), preferably 0.1 to 10% and in particular 0.2 to 5%, expressed in terms of the weight of the dried extract.

The composition for external application to the skin which comprises the extract of Ulva pertusa as an essential component may further comprise, in addition to the essential component, other ingredients commonly used in drugs externally applied, for instance, surfactants, oil components, alcohols, humectants, thickening agents, preservatives, antioxidants, chelating agents, pH-adjusting agents, perfumes, color additives, UV absorbers, UV-scattering agents, vitamins, amino acids and water.

Specific examples of surfactants include nonionic surfactants such as glyceryl monostearate and polyoxyethylene hydrogenated castor oil; anionic surfactants such as sodium stearate and sodium N-acylglutamate; cationic surfactants; and amphoteric surfactants.

Specific examples of oil components include plant oils, animal fats and oils, waxes, hydrocarbons, natural and synthetic fatty acids, natural and synthetic higher alcohols and esters.

The arbitrary components are not restricted to these specific ones. A variety of compositions for external application to the skin can be prepared by appropriately combining the foregoing essential component and arbitrary components. For instance, the composition for external application to the skin comprises 0.01 to 30% of the essential component and, as arbitrary components, 0 to 80% of oil components, 0 to 12% of a surfactant, 1 to 15% of a humectant, a trace amount of preservative and the balance of water. The composition for external application to the skin may be used in any form such as creams, milky lotions, toilet waters, beauty essences, packs, under-makeups, foundations, jellies and ointments.

When the extract of the invention is used in cosmetics for the skin, the cosmetic compositions comprise 0.1 to 10% of the essential component, 20 to 70% of oil components, 2 to 7% of surfactants, 1 to 10% of humectants, trace amounts of preservatives and perfumes and the balance of water for creams applied to the skin; 0.1 to 10% of the essential component, 10 to 40% of oil components, 0 to 15% of alcohols, 1 to 5% of surfactants, 1 to 10% of humectants, 0 to 2% of thickening agents, trace amounts of preservatives and perfumes and the balance of water for milky lotions; 0.1 to 10% of the essential component, 5 to 20% of alcohols, 0 to 2% of surfactants, 2 to 8% of humectants, 0 to 2% of thickening agents, 0 to 0.5% of antioxidants, 0 to 0.1% of chelating agents, 0 to 0.2% of pH-adjusting agents, 0 to trace amount of color additives, trace amounts of preservatives and perfumes and the balance of water for toilet waters and beauty essences; 0.1 to 10% of the essential component, 2 to 10% of alcohols, 2 to 10% of humectants, 0 to 20% of inorganic powder, 10 to 20% of film-forming agents, trace amounts of preservatives and perfumes and the balance of water for packs.

The detail of the mechanism through which the essential component of the present invention shows an excellent effect of inhibiting the aging of the skin has not yet clearly been elucidated, but it is assumed that a biological hyaluronic acid synthesis-promoting substance included in the extract of Ulva pertusa would activate the hyaluronic acid synthesis-ability of the skin cells, that this leads to the improvement in the moisture retention, flexibility and resiliency of the skin and that the essential component thus exhibits a significant effect of inhibiting the aging of the skin.

Moreover, it has been confirmed that the essential component is highly stable in the preparations of agents externally applied to the skin and thus is of much practical use.

The acute toxicity, skin irritation, skin sensitization or the like of the foregoing essential component was examined to confirm the safety thereof and it was found that the component does not lead to practical problems and is highly safe.

The promotor of the present invention exhibits a substantially excellent biological hyaluronic acid synthesis-promoting effect and accordingly activates the skin cells, prevents the aging thereof and the formation of wrinkles and makes the skin smooth and fresh. Thus, the present invention can provide a highly safe composition for external application to the skin.

Consequently, the composition for external application to the skin in which the effective component of the present invention is incorporated can he widely used in a variety of preparations such as various cosmetic creams, milky lotions, toilet waters, beauty essences, packs, under-makeups, foundations, jellies and ointments.

The present invention will be explained in more detail with reference to the following Examples.

### Example 1

Dried Ulva pertusa (100 g) was immersed in 10 volumes of a 30% aqueous ethanol solution at room temperature overnight to give an extract. This process was repeated twice. Then the resulting extracts were combined, concentrated and lyophilized to give 25 g of dried extract of Ulva pertusa. Seaweeds other than Ulva pertusa (Sargassum ringgoldianum, Laminaria japonica, Sargassum fulvellum, Ecklonia cava and Monostroma nitidum) were treated in the same manner as used above to give dried extracts of each.

Then kelatinocytes were separated from the skin of a neonatal (three-day-old) rat by treatment with trypsin, followed by cultivation of the kelatinocytes in a medium for proliferation and cultivation thereof in a medium for differentiation for two days. These cells were treated with each of the foregoing seaweed's extracts in a concentration ranging from 20 µg/ml to 500 µg/ml for 48 hours, then ³H-glucosamine (5 µCi/dish) was added and each culture was further cultivated for additional 24 hours. Further, the culture medium was treated with Actinase to digest the proteins present therein, the hyaluronic acid-containing ³H-GAG (glucosaminoglucan) thus released in the culture medium was separated by the cetyl pyridium chloride precipitation method to determine the radioactivity thereof and the GAG synthesis-promoting ability was calculated in terms of relative value (ratio relative to the control: magnification) with respect to the radioactivity of the control (= 1). The results obtained are listed in the following Table 1.

GAG synthesis-promoting ability (magnification) = A/B
A: Radioactivity of ³H-GAG observed in the presence of each seaweed extract (dpm).
B: Radioactivity of ³H-GAG observed in the absence of each seaweed extract (dpm).

As seen from the results shown in Table 1, brown algae (Phaeophyceae) such as Laminaria japonica, Sargassum fulvellum, Ecklonia cava and Sargassum ringgoldianum; and green algae (Chlorophyceae) such as Monostroma nitidum did not show any GAG synthesis-promoting effect.

On the contrary, the extract of Ulva pertusa promoted the GAG synthesis depending on the concentration thereof and in particular the effect observed at a high concentration was at least two times greater than that of the control.

**Table 1**

| GAG Synthesis-Promoting Effect (Ratio Relative to Control) | | | |
|---|---|---|---|
| Seaweed Extract | Concentration | | |
| | 20 µg/ml | 100 µg/ml | 500 µg/ml |
| Sargassum ringgoldianum | 0.56 | 0.36 | 0.23 |
| Laminaria japonica | 0.99 | 0.92 | 0.98 |
| Sargassum fulvellum | 0.97 | 0.99 | 1.03 |
| Ecklonia cava | 1.01 | 1.05 | 0.99 |
| Ulva pertusa | 1.19 | 1.49 | 2.04 |
| Monostroma nitidum | 1.02 | 1.07 | 1.04 |

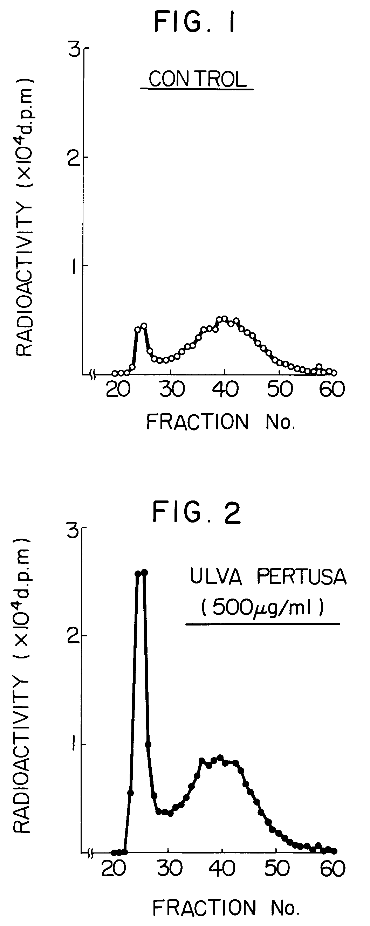

Then the total GAG obtained from the control (free of seaweed extract) and the culture medium in which the cells were cultivated in the presence of the extract of Ulva pertusa (500 µg/ml) were chromatographed on a Sepharose CL-6B column (1.1 cmφ × 60 cm) and eluted with an eluant (50 mM-Tris HCl buffer containing 0.15 M-NaCl; pH 7.4) at room temperature and a flow rate of 0.37 ml/min. Fractions (1.04 ml each) were collected and the radioactivities thereof were determined to calculate radioactivities of hyaluronic acid and sulfated GAG present in the total GAG. The radioactivities of these fractions are shown in Figs. 1 and 2. Among these fractions, Fraction Nos. 22 to 29 are hyaluronic acid-containing fractions and Fraction Nos. 30 to 57 are sulfated GAG-containing fractions. Table 2 shows the total amount of hyaluronic acid and sulfated GAG as well as the ratio relative to the control. As seen from the data given in Table 2, when the cells were treated with 500 µg/ml of the Ulva pertusa extract, the hyaluronic acid content in the total GAG increased up to 4.8 times that of the control. This clearly indicates that the Ulva pertusa extract exhibits a high epidermal hyaluronic acid synthesis-promoting activity.

**Table 2**

| Extract | Total GAG | | Hyaluronic Acid | | Sulfated GAG | |
|---|---|---|---|---|---|---|
| | R.A. (dpm) | Relative Ratio | R.A. (dpm) | Relative Ratio | R.A. (dpm) | Relative Ratio |
| Control | 83905 | -- | 15525 | -- | 68380 | -- |
| Ulva pertusa (500 µg/ml) | 205161 | 2.45 | 74104 | 4.77 | 131052 | 1.92 |
| R.A.: Radioactivity | | | | | | |

### Example 2

A hydrophilic ointment having a composition detailed in the following Table 3 (containing 5% of each substance to be tested) was prepared and applied, one time per day over 7 days, onto the skin on the back of groups (comprising 6 animals each) of hairless mice in an amount of 0.2 g. These animals were sacrificed, the epidermis thereof was separated from the portion to which the ointment had been applied, defatted and dehydrated and then treated with Actinase. After removal of proteins present, the amount of the hyaluronic acid separated was quantitatively analyzed by the carbazole-sulfuric acid method. The results obtained are listed in the following Table 4. In the foregoing test, the extracts prepared in Example 1 were used as the substances to be tested. In addition, the amount of hyaluronic acid is expressed in terms of relative value of the weight thereof (average) per unit area of the epidermal tissue with respect to that of the control (free of substance to be tested).

**Table 3**

| Component | Amount (% by weight) |
|---|---|
| White vaseline | 25.0 |
| Stearyl alcohol | 20.0 |
| Polyoxyethylene (60) hydrogenated castor oil | 4.0 |
| Glyceryl monostearate | 1.0 |
| Propylene glycol | 12.0 |
| Methylparaben | 0.1 |
| Propylparaben | 0.1 |
| Substance to be tested | 5.0 |
| Purified water | balance |

**Table 4**

| Substance to be Tested | Amount of Hyaluronic Acid (relative value) |
|---|---|
| Control (free of Substance to be tested) | 100 |
| Extract of Ulva pertusa | 130 |
| Extract of Monostroma nitidum | 101 |
| Extract of Sargassum ringgoldianum | 98 |
| Extract of Laminaria japonica | 100 |
| Extract of Sargassum fulvellum | 99 |
| Extract of Ecklonia cava | 98 |

The results listed in Table 4 clearly indicate that the group of mice to which the hydrophilic ointment comprising the extract from Ulva pertusa as an effective component was applied had a hyaluronic acid content in the epidermis higher than that of the control group.

On the other hand, there was not observed any significant increase in the hyaluronic acid contents in the groups to which hydrophilic ointments comprising the extracts other than that of Ulva pertusa were applied.

### Example 3

The control group and Ulva pertusa extract-applied group (comprising 6 animals each) which had been examined in the same manner used in Example 2 were sacrificed to prepare tissue sections of the epidermis to which the ointments had been applied, followed by subjecting these sections to hematoxylin-eosin staining and examination of the structures of the skin.

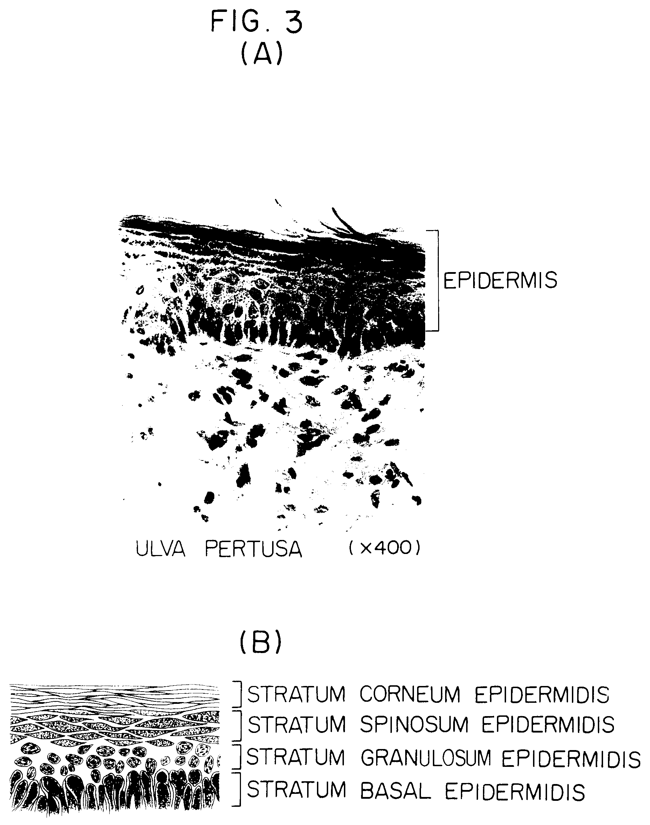

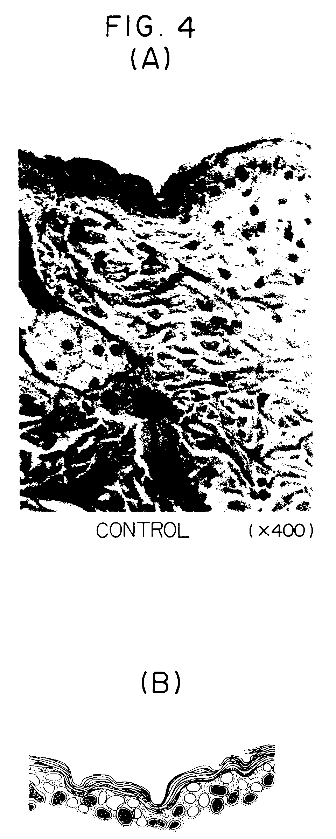

As a result, it was found out that the epidermal tissues of each subject belonging to the group to which the extract of Ulva pertusa had been applied had complete epidermal tissues and had been activated as compared with those of the control group and, in particular, that the kelatinocytes in the epidermal basal layer are regularly arranged. A typical example thereof is shown in the attached Fig. 3 and the control is shown in Fig. 4.

The foregoing results indicate that the application of a hydrophilic ointment comprising the extract of Ulva pertusa as the effective component used in the invention can activate the epidermal kelatinocytes, promote the hyaluronic acid synthesis and consequently prevent the aging of the epidermal tissues.

### Example 4

To 3.0 kg of dried Ulva pertusa were added 20 volumes of water relative to 1 volume of the dried Ulva pertusa and stirred at 5 °C for 24 hours to perform an extraction. This procedure was repeated twice. The extracts thus obtained were combined and concentrated using an evaporator under reduced pressure. Then the concentrate was passed through an ultrafiltration membrane (Amicon YM100) having a fractional molecular weight of 100,000 to give a fraction comprising components having molecular weights of not more than 100,000. This fraction was then passed through another ultrafiltration membrane (Amicon YM3) having a fractional molecular weight of 3,000 to remove a fraction comprising components having molecular weights of not more than 3,000 and to thus give a fraction comprising components having molecular weights ranging from 3,000 to 100,000 and then the fraction was lyophilized to give 150 g of a dried Ulva pertusa extract.

Then components 1 to 7 and components 8 to 11 listed in Table 5 were separately mixed and dissolved, followed by addition of the solution of the components 1 to 7 to the solution of the components 8 to 11 with stirring to form an emulsion and addition of component 12 in the course of cooling of the emulsion to room temperature to give a cream shown in Table 5. Numerical values each in Table 5 represents added amount (% by weight) (those in the following Tables are shown in the same way also).

**Table 5**

| Component | | Present Invention | Comparative Ex. |
|---|---|---|---|
| 1. | liquid paraffin (# 70) | 5.0 | 5.0 |
| 2. | Squalane | 14.0 | 14.0 |
| 3. | Cetostearyl alcohol | 6.0 | 6.0 |
| 4. | Bees wax | 2.0 | 2.0 |
| 5. | Glyceryl monostearate | 2.0 | 2.0 |
| 6. | POE(20) sorbitan monolaurate | 2.0 | 2.0 |
| 7. | Propylparaben | 0.1 | 0.1 |
| 8. | Ulva pertusa extract (dried product) | 0.5 | --- |
| 9. | Diglycerin | 5.0 | 5.0 |
| 10. | Methylparaben | 0.2 | 0.2 |
| 11. | Purified water | balance | balance |
| 12. | Perfume | trace amount | trace amount |

The effectiveness of the cream thus prepared was determined as follows.

Statistically equivalent women (30 to 50-year-old) were selected, the cream of the present invention (A) and a comparative cream (B) were continuously applied to the faces, twice a day (in the morning and at night) over 3 months, according to the half-face method in which the cream of the invention and the comparative cream were applied onto the right and left halves of the face of each subject, respectively. Thus, the degree of improvement in fine wrinkles and condition of the skin (glossiness, moistness) was evaluated. The results obtained are summarized in Table 6.

**Table 6**

| Evaluated Item | A >> B | A > B | A ≒ B | B > A | B >> A |
|---|---|---|---|---|---|
| Improvement of Fine Wrinkles | 3 | 7 | 8 | 2 | 0 |
| Improvement of Skin Condition | 5 | 8 | 6 | 1 | 0 |
| A>>B: The cream of the present invention is greatly superior to the comparative cream; A>B : The cream of the present invention is better than the comparative cream; A ≒ B: The cream of the present invention is almost identical with the comparative cream; B>A : The comparative cream is better than the cream of the present invention; B>>A: The comparative cream is greatly superior to the cream of the present invention. | | | | | |

As seen from the results shown in Table 6, it was found that the cream of the present invention which comprises the Ulva pertusa extract exhibits effects of improving fine wrinkles and condition of the skin greater than those of the comparative cream free of the Ulva pertusa extract.

The foregoing results clearly indicate that external application of the cream comprising the effective component of the present invention significantly promotes the hyaluronic acid synthesis capacity of the epidermal cells and substantially eliminates the aging symptoms of the skin. No abnormalities such as erythema on the skin during and after the use of the cream of the present invention over 3 months were observed.

### Example 5

Components 1 to 6 listed in the following Table 7 were, in order, added to and dissolved in component 7 and then component 8 was added to the resulting mixture to give toilet waters shown in Table 7.

**Table 7**

| Component | Present Invention | Comparative Ex. |
|---|---|---|
| 1. Ulva pertusa extract (dried) | 0.1 | --- |
| 2. 1,3-butylene glycol | 3.0 | 3.0 |
| 3. citric acid | 0.02 | 0.02 |
| 4. sodium citrate | 0.05 | 0.05 |
| 5. ethanol | 18.0 | 18.0 |
| 6. methylparaben | 0.1 | 0.1 |
| 7. purified water | balance | balance |
| 8. perfume | trace amount | trace amount |

Incidentally, the Ulva pertusa extract used herein as component 1 was the product obtained in Example 4 (this product is also used in the following Examples).

The effectiveness of the toilet waters (C: present invention; D: Comparative Example) thus prepared was examined in the same manner and evaluation standard used in Example 4. The results obtained are summarized in the following Table 8.

**Table 8**

| Evaluated Item | C >> D | C > D | C ≒ D | D > C | D >> C |
|---|---|---|---|---|---|
| Improvement of Fine Wrinkles | 2 | 8 | 8 | 2 | 0 |
| Improvement of Skin Condition | 3 | 8 | 7 | 2 | 0 |

As seen from the results shown in Table 8, it was found that the toilet water of the present invention which comprises the Ulva pertusa extract exhibits effects of improving fine wrinkles and condition of the skin greater than those of the comparative toilet water free of the Ulva pertusa extract.

The foregoing results clearly indicate that external application of the toilet water comprising the effective component of the present invention significantly promotes the hyaluronic acid synthesis capacity of the epidermal cells and substantially improves aging symptoms of the skin. No abnormalities such as erythema on the skin during and after the use of the toilet water of the present invention over 3 months were observed.

### Example 6

Components 1 to 4 and components 5 to 9 listed in Table 9 were separately dissolved and then mixed together to give beauty essences.

**Table 9**

| Component | Present Invention | Comparative Ex. |
|---|---|---|
| 1. Ulva pertusa extract (dried) | 1.0 | --- |
| 2. glycerin | 4.0 | 4.0 |
| 3. carboxyvinyl polymer | 0.5 | 0.5 |
| 4. purified water | balance | balance |
| 5. dℓ-α-tocopheryl acetate | 0.1 | 0.1 |
| 6. ethanol | 10.0 | 10.0 |
| 7. POE(40) hydrogenated castor oil | 0.5 | 0.5 |
| 8. methylparaben | 0.1 | 0.1 |
| 9. perfume | trace amount | trace amount |

The effectiveness of the beauty essences (E: present invention; F: Comparative Example) thus prepared was examined in the same manner and evaluation standard used in Example 4. The results obtained are summarized in the following Table 10.

**Table 10**

| Evaluated Item | E >> F | E > F | E ≒ F | F > E | F >> E |
|---|---|---|---|---|---|
| Improvement of Fine Wrinkles | 4 | 9 | 6 | 1 | 0 |
| Improvement of Skin Condition | 6 | 8 | 6 | 0 | 0 |

As seen from the results shown in Table 10, it was found that the beauty essence of the present invention which comprises the Ulva pertusa extract exhibits effects of improving fine wrinkles and condition of the skin greater than those of the comparative beauty essence free of the Ulva pertusa extract.

The foregoing results clearly indicate that external application of the beauty essence comprising the effective component of the present invention significantly promotes the hyaluronic acid synthesis capacity of the epidermal cells and substantially eliminates the aging symptoms of the skin. No abnormalities such as erythema on the skin during and after the use of the beauty essence of the present invention over 3 months were observed.

### Example 7

Components 1 to 7 listed in Table 11 were dissolved under heating at 70 °C. Separately, components 8 to 13 were heated to 70°C to dissolve them, followed by addition of the oils solution (components 1 to 7) to the dissolved components 8 to 13 to emulsify them and addition of component 14 in the course of cooling the resulting emulsion to room temperature to give milky lotions shown in Table 11.

**Table 11**

| Component | | Present Invention | Comparative Ex. |
|---|---|---|---|
| 1. | liquid paraffin (#70) | 10.0 | 10.0 |
| 2. | isopropyl myristate | 1.5 | 1.5 |
| 3. | glyceryl monostearate | 0.5 | 0.5 |
| 4. | stearic acid | 2.0 | 2.0 |
| 5. | POE(20) stearyl ether | 0.7 | 0.7 |
| 6. | glycyrrhetinic acid | 0.1 | 0.1 |
| 7. | butylparaben | 0.1 | 0.1 |
| 8. | Ulva pertusa extract (dried) | 0.05 | --- |
| 9. | glycerin | 2.0 | 2.0 |
| 10. | carboxyvinyl polymer (Mw 1,000,000∼1,500,000) | 0.1 | 0.1 |
| 11. | ethanol | 10.0 | 10.0 |
| 12. | methylparaben | 0.1 | 0.1 |
| 13. | purified water | balance | balance |
| 14. | perfume | trace amount | trace amount |

The effectiveness of the milky lotions thus prepared was examined in the same manner and evaluation standard used in Example 4 and there were observed effects of improving fine wrinkles and skin condition almost identical to those obtained in Example 4.
No abnormalities such as erythema on the skin during and after the use of the milky lotion of the present invention over 3 months were observed.

## Claims

1. A promotor for biological hyaluronic acid synthesis comprising as an effective component an extract of the seaweed Ulva pertusa.

2. The promotor of claim 1 wherein the extract of Ulva pertusa is obtained by extracting Ulva pertusa with water, a hydrophilic solvent or a mixture thereof.

3. The promotor according to claim 1 or 2, wherein the extract of Ulva pertusa is fractionated and includes components having molecular weights ranging from 3,000 to 100,000.

4. A composition for external application to the skin comprising an extract of the seaweed Ulva pertusa and an inert carrier and/or diluent.

5. The composition of claim 4 wherein the extract of Ulva pertusa is obtained by extracting Ulva pertusa with water, a hydrophilic solvent or a mixture thereof.

6. The composition of claim 4 or 5 wherein the extract of Ulva pertusa is fractionated and includes components having molecular weights ranging from 3,000 to 100,000.

7. The composition of any one of claims 4 to 6 which comprises the extract of Ulva pertusa in an amount ranging from 0.01 to 30% by weight as expressed in terms of the weight of dry powder thereof.

8. The composition of claim 7 which comprises the extract of Ulva pertusa in an amount ranging from 0.1 to 10% by weight as expressed in terms of the weight of dry powder thereof.

9. The composition of claim 8 wherein the amount of the extract of Ulva pertusa ranges from 0.2 to 5% by weight as expressed in terms of the weight of dry powder thereof.

10. The composition of any one of claims 4 to 9 which further comprises a surfactant.

11. The composition of any one of claims 4 to 9 which further comprises a humectant.

12. Use of an extract of the seaweed Ulva pertusa for the preparation of a promotor for biological hyaluronic acid synthesis comprising the extract as an effective component.

13. Use of an extract of the seaweed Ulva pertusa for the preparation of a composition for external application to the skin comprising the extract and an inert carrier and/or diluent.

14. The use of claim 12 or 13 wherein the extract of Ulva pertusa is obtained by extracting Ulva pertusa with water, a hydrophilic solvent or a mixture thereof.

15. The use of any one of claims 12 to 14 wherein the extract of Ulva pertusa is fractionated and includes components having molecular weights ranging from 3,000 to 100,000.

## Claims (Claims for the following Contracting State(s): ES)

1. A process for preparing a promotor for biological hyaluronic acid synthesis comprising as an effective component an extract of the seaweed Ulva pertusa, which comprises optionally drying the seaweed, extracting Ulva pertusa, preferably with water, a hydrophilic solvent or a mixture thereof, and optionally fractionating the extract.

2. The process according to claim 1, wherein the fractionated extract includes components having molecular weights ranging from 3,000 to 100,000.

3. A process for the preparation of a composition for external application to the skin comprising an extract of the seaweed Ulva pertusa which comprises combining an extract obtained according to claim 1 or 2 and an inert carrier and/or diluent.

4. The process according to claim 3 wherein the composition comprises the extract of Ulva pertusa in an amount ranging from 0.01 to 30% by weight as expressed in terms of the weight of dry powder thereof.

5. The process according to claim 4 wherein the composition comprises the extract of Ulva pertusa in an amount ranging from 0.1 to 10% by weight as expressed in terms of the weight of dry powder thereof.

6. The process according to claim 5 wherein the amount of the extract of Ulva pertusa ranges from 0.2 to 5% by weight as expressed in terms of the weight of dry powder thereof.

7. The process according to any one of claims 3 to 6 wherein the composition is further combined with a surfactant.

8. The process according to any one of claims 3 to 6 wherein the composition is further combined with a humectant.
